# EUROPEAN PATENT SPECIFICATION

(11) **EP 0 613 355 B1**
(45) Date of publication and mention of the grant of the patent: **22.01.1997**
(21) Application number: 92912740.5
(22) Date of filing: 27.05.1992
(51) Int. Cl.: A61F 5/44, A61F 5/455

(54) **IMPROVED PAD AND PAD TUBE CONNECTOR FOR THE MANAGEMENT OF URINARY INCONTINENCE**
UNTERLAGE FÜR DIE BEHANDLUNG VON URININKONTINENZ
ELEMENT ABOSRBANT ET RACCORD DE TUBE D'ELEMENT ABSORBANT AMELIORES PERMETTANT DE GERER L'INCONTINENCE URINAIRE

(30) Priority: 20.11.1991 US 795322
(43) Date of publication of application: 07.09.1994
(73) Proprietor: UROHEALTH, Inc. (California), Newport Beach, California 92660 (US)
(72) Inventor: KUNTZ, David H., Los Angeles, CA 90049 (US); ELSON, Edward E., Anaheim, CA 92807 (US)
(74) Representative: Maggs, Michael Norman
(86) International application number: US9204464
(87) International publication number: WO9309736

(56) References cited:
- FR-A- 1 485 683
- FR-A- 2 534 470
- GB-A- 2 148 126
- US-A- 4 246 901
- US-A- 4 747 166

## Description

The present invention relates to absorptive aspiratable pads for use in urine collection and disposal systems and, more particularly, to an improved pad which is more efficient in the urine collection and disposal task.

Incontinence in wheelchair and bed patients, whether in hospitals, nursing homes, or at home, presents a particular and continuing problem with respect to the care of such patients. Bodily incontinence is even more prevalent among the elderly. For anatomical reasons, the design of urine collection appliances for women is particularly difficult.

In an effort to keep bed patients' bedding clean and dry so that the patient may be as comfortable as possible, it is frequently necessary to change the bedding several times a day for a patient having urinary incontinence. After a urinary discharge, there is a delay before the attendant becomes aware of the soiled and wet bedding so that steps can be taken to replace it. It is during this time that the patient is in contact with the urine-soaked bedding and is most prone to develop a rash or sores due to such exposure. Similar problems arise with respect to incontinent patients confined to wheelchairs.

In the above-mentioned circumstances of a patient lacking the requisite mobility or self-management of the effects of their incontinence, the use of disposable absorbent pads of some kind have been previously employed. There are many such disposable pads commercially available for this purpose, such as pads normally involving an absorbent core, typically a cellulose wadding or other hydroscopic material. The problem with pads of this construction is their inability to receive and retain urine at significantly high flow rates or volumes without leakage. In addition, the pads are typically bulky, either when wet from use or in the dry state, and still effectively require the need for repeated replacement, at considerable inconvenience, and, therefore, cost to those in attendance with the patient. Replacement of the urine soaked bulky pads involves handling the pads, which is an unpleasant task. Further, repeated replacement is also associated with the inconvenience of storing a great number of pads to be ready for use, and the disposal of a large number of pads, creating a volumetric disposal problem.

Earlier attempts at dealing with the urinary incontinence problem include U.S. Patents Nos. 3,349,768, 4,246,901, 4,610,675, 4,713,065, 4,200,102 and 4,886,508, and GB-A-2,148,126 and FR-A-1,485,683.

US-A-4246901 is directed to a urine collection device which incorporates a wicking material in a collection chamber. In particular, Figure 4 of this patent shows a device including a first body of wicking material within a chamber, the wicking material being brought into contact with the urethra. The chamber includes a conduit portion which is disposed within a second body of wicking material filling a reservoir. The reservoir further includes a tube, filled with a one-way wicking material, which is, in turn, connected to a collection pouch.

A more recent method of dealing with urinary incontinence is disclosed in US-A-4,747,166, entitled "Fluid Aspiration System for the Management of Urinary Incontinence" on which the precharacterising portion of Claim 1 is based. The aspiration system disclosed therein includes a vacuum pump and urine collection reservoir connected via a tube to a pad composed of absorbent material suitable to collect urine. In a first embodiment, the tube is attached, through the use of a connector, to an external opening of a central bore extending through the absorbent pad. In a second embodiment, the tube is insertable into the central bore. However, in neither instance is there a wicking assembly attached to the tube to aid in drawing fluid from the absorbent material into the tube. The pad disclosed therein has a series of layers of highly absorbent cellulose tissue. The tissue layers are surrounded at the bottom by a thin impermeable layer and at the top by a permeable layer, the two layers being joined at the middle edge of the pad. A sealing ring was provided to sealably encompass the genital area to provide sealing there.

It is an object of the invention to provide an improved design of an absorbent pad, utilizable with an aspiration system, which has a collection structure within the pad which will contribute to the even drainage of the pad and reduce unwanted collection at points within the pad volume.

According to the present invention an absorptive aspiratable pad of the type comprising an elongate fluid impermeable lower liner, defining a planar surface forming the bottom and at least a portion of the sides of the pad, bulk absorbent material supported by and co-extensive with the impermeable lower liner and covered by a layer of non-wetting permeable material, which substantially envelopes the lower liner, the bulk absorbent material and an elongate tube having a multiplicity of perforations situated within the permeable material and in fluid contact with the bulk absorbent material is characterised in that a first layer of wicking material is attached to the elongate tube and extends substantially the length of and in contact with the bulk absorbent material and that a second layer of wicking material is attached to the elongate tube and to the first layer of elongate wicking material, whereby the first and second layers of wicking material and the elongate tube form a wicked tube assembly.

The bulk absorbent material preferably substantially covers the length of the wicked tube assembly.

The elongate tube and the first and second layers of wicking material may lie outside of and adjacent the bulk absorbent material opposite the elongate fluid impermeable lower liner or outside of and adjacent the bulk absorbent material and adjacent the elongate fluid impermeable lower liner or substantially within the bulk absorbent material.

Other aspects, features and advantages of the invention, its configuration, construction, and operation will be best understood from the following detailed description, taken in conjunction with the accompanying drawings, in which:
Figure 1 illustrates an aspiration system employing an absorbent pad in accordance with the present invention and a pad tube connector, shown in perspective, connected to a generalized urine collection reservoir, shown in generalized section, and connected to a vacuum pump which is schematically illustrated;
Figure 2 is a perspective cutaway view of the pad of Figure 1 illustrating its layering of materials;
Figure 3 is a cross-section of a first embodiment of the pad of Figure 1 taken along line 3-3 of Figure 1;
Figure 4 is a cross-section of a second embodiment of the pad of Figure 1 taken along line 3-3 of Figure 1;
Figure 5 is a cross-section of a third embodiment of the pad of Figure 1 taken along line 3-3 of Figure 1;
Figure 6 is an expanded cross-sectional view of the wicking structure shown in Figures 2-5;
Figure 6a is an expanded cross-sectional view of the wicking structure shown in Figure 6, but illustrating a petal shaped void;
Figure 7 is a side sectional view of a two-piece connector having an ejection mechanism;
Figure 8 is a side sectional view of one-piece of the two-piece connector of the embodiment of Figure 7 shown in relation to a tube;
Figure 9 is a side sectional view of one-piece of the two-piece connector of the embodiment of Figures 7 and 8 shown connected to a tube;
Figure 10 is a side sectional view of the first piece of the two-piece connector being fitted with its associated second piece;
Figure 11 is a side sectional view of the connector in assembled and partially retracted position;
Figure 12 is a side sectional view of the connector of Figures 7 and 11 fully engaging a dry absorbent pad;
Figure 13 is a side sectional view of the connector of Figure 12 in a position partially ejecting a wet absorbent pad;
Figure 14 is a side sectional view of the connector of Figures 12 and 13 in a position fully ejecting a wet absorbent pad;

### DETAILED DESCRIPTION OF THE PREFERRED EMBODIMENT

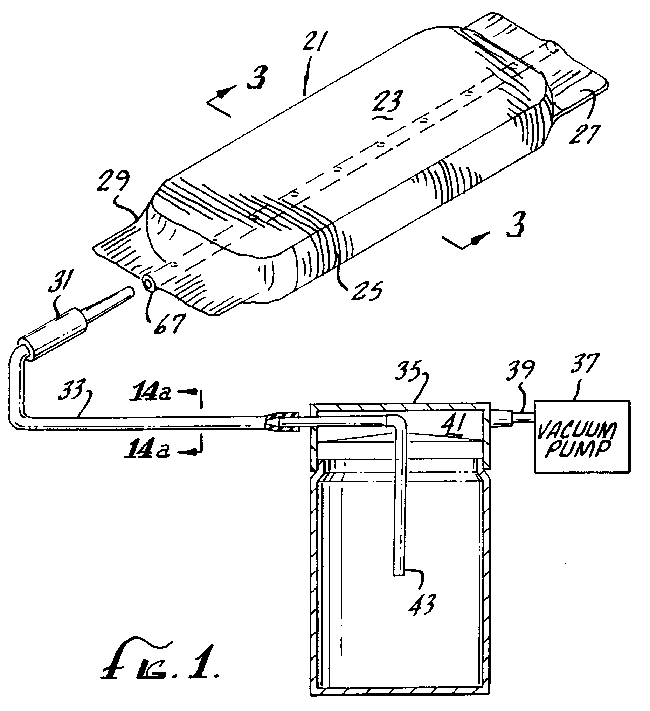

Referring to Figure 1, a generally rectangular absorptive pad 21 has an upper surface 23. The absorptive pad 21 is somewhat similar in overall external appearance, and is about the same size and shape as commercially available sanitary napkins. Absorptive pad 21 is intended to make direct contact with a patient's body surrounding the urethral opening, and is easily positionable against the patient, especially between the patient's body and garments. Absorptive pad 21 is flexible in all directions along its upper surface 23, and is, therefore, easily fittable with all patients.

The exterior of the absorptive pad 21 is covered with an essentially non-wetting permeable material 25, which envelops the absorptive pad 21, along its length and is sealed at one end 27, hereafter referred to the closed end 27, and may be partially or totally closed at the other end 29, hereafter referred to as the tube end 29, resulting in the contents of the absorptive pad 21 being sealably surrounded by the essentially non-wetting permeable material 25.

An improved connector 31 removably couples a tube 33 to tube end 29 of absorptive pad 21. The other end of tube 33 is connected to a urine collection vessel 35. Urine collection vessel 35 is further connected to a vacuum pump 37, or similar pressure reducing device, via a tube 39, and is configured to collect urine through a pressure reduction action, and to isolate vacuum pump 37 from any liquids flowing through tube 33. Urine collection vessel 35 is configured to permit tube 39 to draw air from a space 41 at the upper portion of urine collection vessel 35, creating a reduced pressure, and causing delivery of a mixture of urine and air through tube 33, and through a downcomer tube 43 directed into the urine collection vessel 35.

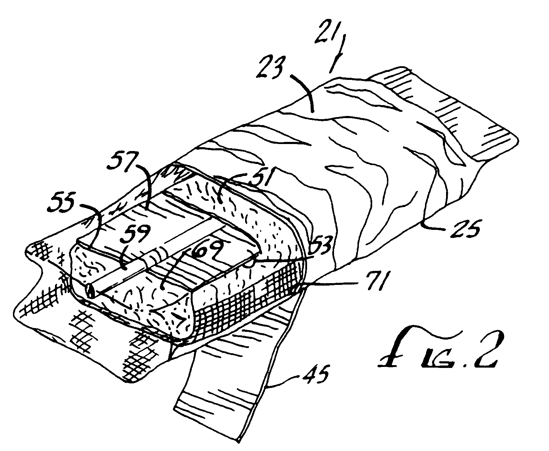

Figure 2 is a partially cut away view of the preferred embodiment of the pad of the present invention. At the bottom of pad 21 is an adhesive layer having a plastic or other non-sticking tear-away strip 45 which may be removed to expose the adhesive layer. The adhesive layer, to be shown later, is attached to the outer permeable non-wetting layer 25 and facilitates the fixation of the pad 21 to the inner surfaces of the user's garments, diaper or covering, when the pad 21 is utilized and held in place with a garment, diaper or covering. At the outer periphery, the permeable non-wetting layer 25 is illustrated as surrounding all of the layers in absorbent pad 21. At the top 23 of the absorbent pad 21, and adjacent and inward of the permeable non-wetting layer 25, is a highly absorbent inner layer 51 which extends continuously enclosing an inner volume of the absorbent pad 21, and within the boundary of the permeable non-wetting layer 25.

Near the top 23 of absorbent pad 21, and adjacent and below the highly absorbent inner layer 51, is a structure collectively referred to as a wick tube assembly 53. Wick tube assembly 53 is made up of at lest a first layer of wicking material 55 and at least a second layer of wicking material 57 sandwiching a tube 59. The tube 59 is perforated, as will be shown later, and will hereinafter be referred to as perforated tube 59. Generally, wicking materials 55 and 57 are, before being brought together to form wick tube assembly 53, generally planar, porous sheets of material. In the referenced embodiment wicking materials 55 and 57 have a paper towel like consistency. The wicking materials 55 and 57 must be absorbent enough to attract liquid and transmit liquids through wicking action, but not retentive of liquid in a manner which would inhibit wicking. The sheets of material forming the wicking materials 55 and 57 are typically glued or otherwise fixed to each other, and to perforated tube 59, over the bulk of their contacting surfaces. This ensures that air is not drawn into the perforated tube 59 through the boundary layer between noncontacting layers. In Figure 2, both of the layers of the wicking materials 55 and 57 are shown as being coextensive with each other. However, one of the layers of the wicking materials 55 and 57 may be abbreviated in width. Wicking material layers 55 and 57 may have similar or dissimilar absorptive characteristics.

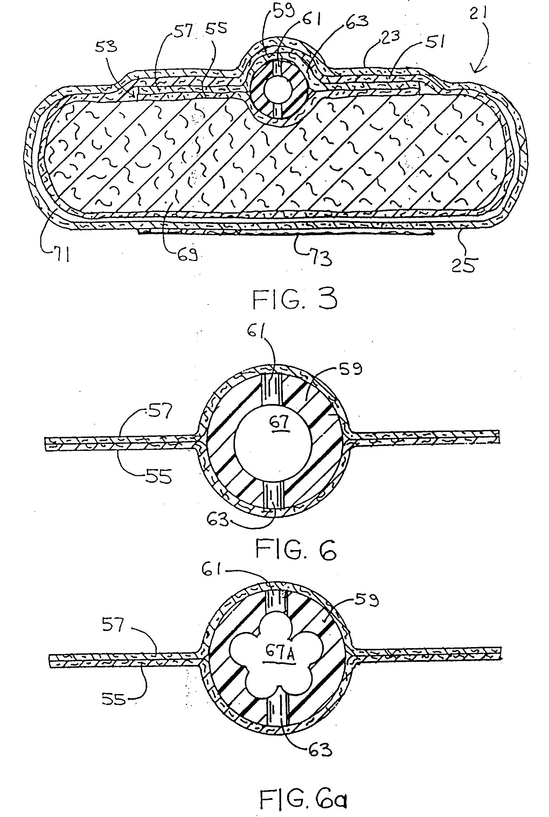

Figure 3 is a cross-section taken along line 3-3 of Figure 1, and is essentially equivalent to a cross-section taken at any point along the length of the central portion of the absorbent pad 21. Now referring to both Figures 2 and 3, note that the wicking materials 55 and 57 conform closely and are preferably adhered to the outer surface of the perforated tube 59, to ensure efficient operation. This close conformance ensures that there will be no volumes of space of a size where liquids will collect rather than be wicked. Nor will there exist a channel that serves as a shunt for air to flow as this would decrease the effectiveness of the wick in drawing liquid from its perimeter and from the inner volume of pad 21.

Note, that perforated tube 59 has a linear bore formed therethrough and is provided with one or more apertures, namely generally upper aperture 61 and generally lower aperture 63. The apertures are most easily formed by punching or drilling a single bore through perforated tube 59. A series of generally upper and lower apertures 61 and 63 are formed throughout the length of perforated tube 59 residing within absorbent pad 21. The perforated tube is sealed closed at one end.

Typically, the lengths of perforated tube 59 which are utilized within absorbent pad 21 are formed by punching or drilling straight through the perforated tube 59 at intervals. The axes of the multiple bores of the upper and lower apertures 61 and 63 may lie within the same plane, such that all upper apertures 61 are at the uppermost surface of the perforated tube 59, and all lower apertures are at the lowermost surface of the perforated tube 59. Perforated tube 59 may be sandwiched between the wicking layers 55 and 57 with apertures 61 and 63 in a plane which may vary from perpendicular to parallel to the horizontal axis of the wick tube assembly 53. Perforated tube 59 is usually obtained in lengths which have been machine punched or drilled. Alternatively, the perforated tube 59 may be perforated or punched in the process of assembly of pad 21.

The perforated tube 59 extends for substantially the length of the absorbent pad 21. At the closed end 27 of the absorbent pad 21, the perforated tube 59 is sealed closed at its end. The other end of perforated tube 59, at the tube end of the pad 21 is left open, and terminates at a plane, and has an end profile matching the cross-sectional shape of the sectional view of perforated tube 59 as shown in Figures 1, 2, 3, 4, 5 and 6.

Underneath wick tube assembly 53, and adjacent the highly absorbent inner layer 51 is the bulk absorbent material 69. The bulk absorbent material 69 forms a core of urine absorptive material. This material has the characteristic of rapid absorption and release of urine, and acts as a temporary reservoir for increased holding capacity. Note, that the highly absorbent inner layer 51 completely surrounds the bulk absorbent material 69. Layer 51 although highly absorbent, ready releases liquid to wick tube assembly 53 and bulk absorbent material 69. With this configuration, moisture within bulk absorbent material 69 will readily move into and through the highly absorbent inner layer 51. As layer 51 collects moisture it will be more subject to being withdrawn into the wick tube assembly 53.

An impermeable lower liner 71 is located within the permeable and non-wetting layer 25, and outside the highly absorbent inner layer 51. In this manner, the highly absorbent inner layer 51, will be prevented from wetting the patient's garments or bedding. The impermeable lower liner 71 does not extend circumferentially completely around the highly absorbent inner layer 51, but surrounds inner layer 51 at its bottom and side surfaces. Impermeable lower linear 71 may extend partially or totally upwardly about the sides of pad 21.

At the bottom and side of absorbent pad 21, the impermeable lower liner 71 separates the permeable non-wetting layer 25, and the outside of the highly absorbent inner layer 51. However, at the sides of absorbent pad 21, near the top, the impermeable lower liner 71 ends, and the portions of the permeable non-wetting layer 25, and outside the highly absorbent inner layer 51, which extend over the top 23 of absorbent pad 21, are in contact with each other. An optional adhesive layer 73, referred to earlier, is illustrated at the bottom of the absorbent pad 21, immediately adjacent the permeable non-wetting layer 25. In Figure 3 it is shown without the protective non-sticking tear-away strip 45. Adhesive layer 73 is used to secure absorbent pad 21 to the inside of a patient's clothing, to better secure the absorbent pad 21 to the patient.

A second embodiment of the absorbent pad 21 is shown in Figure 4, wherein the wick tube assembly 53 is located within the bulk absorbent material 69. The other layers, namely permeable non-wetting material 25, highly absorbent inner layer 51 and impermeable liner 71 surround the bulk absorbent material 69 in the same manner as was illustrated in Figure 2.

A third embodiment of the absorbent pad 21 is shown in Figure 5, wherein the wick tube assembly 53 is located beneath the bulk absorbent material 69, and between the highly absorbent inner layer 51 and the impermeable liner 71.

A closer cross-sectional view of the wick tube assembly 53 is illustrated in Figure 6. Note, the extent to which the first layer of wicking material 55 and a second layer of wicking material 57 have the area of one side of their surface completely taken up with attachment, either to the exterior surface of perforated tube 59 or to the opposing surface of each other. The transition from areas of the first and second wicking materials 55 and 57 which are in contact with each other, to the areas of contact of the first and second wicking materials 55 and 57 about the external surface of perforated tube 59, does not create a significant space at the point of transition.

Note, also that the ends of apertures 61 and 63 at the outer surface of perforated tube 59 are completely covered by the material from first and second wicking materials 55 and 57. When apertures 61 and 63 are in planes other than perpendicular to the layers 55 and 57 they are still covered by layers 55 and 57. In this manner, none of the upper and lower apertures 61 and 63 are exposed to the ambient atmosphere, and will always be exposed, when absorbent pad 21 is wet, to wicked liquid urine.

The portions of first and second wicking materials 55 and 57 which adhere to each other and the perforated tube 59, form an extended planar surface which is in contact with other elements of pad 21. When the extended planar wicking surface is directly in contact with other absorbent material, including either bulk absorbent material 69, or highly absorbent inner layer 51, significant frictional resistance to axial and lateral movement is achieved.

The internal portion of the perforated tube 59 will preferably have a circumferentially smooth configuration. The cross-section of the inside of perforated tube 59, appearing in Figures 1, 2, 3, 4, 5 and 6 has a circular-shaped void 67. It is understood that other shapes, including cross-sectional shapes for the void 67 within perforated tube 59 may be utilized, such as that shown in Figure 6a which is more efficient at moving a liquid gas two-phase mixture, and with less noise from gurgling, etc.

All of the embodiments of the improved pad of the instant invention, which were shown in Figures 1-6a are amenable for connection to tube 33 of Figure 1, utilizing any embodiment of the connector, one suitable embodiment of which is illustrated in detail in the remaining figures.

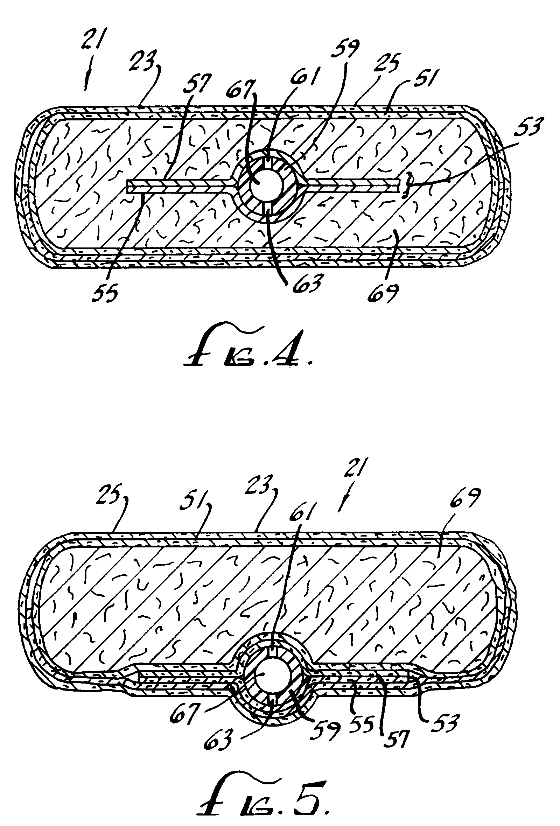

Shown in Figures 7-14 is a two-piece connector 301 embodying an ejection mechanism. The ejection mechanism enables a urine soaked pad 21 to be removed by handling the tube 33 and connector 31 shown in Figure 1. Referring to Figure 7, connector 301 has a cylindrical housing 303 having a small forward aperture 305 and an internal bore 307. A circular inwardly disposed land 309 extends from the surface of the internal bore 307 at the end of cylindrical housing 303 opposite the forward aperture 305.

Internal to the cylindrical housing 303 an internal member 311, having a bore 312, defines an elongate tapering snout 313, an external land 315 which slidably engages the internal bore 307 of cylindrical housing 303, and circular fitting 317 having a frusto-conical surface 319 and reduced outer diameter surface 321.

Internal member 311 is axially slidable within cylindrical housing 303, the areas of contact between the external land 315 and the internal bore 307, and between the small forward aperture 305 and the elongate tapering snout 313 providing axial stability. The internal surface of tube 33 is securely attached over the circular fitting 317, frusto-conical surface 319, and reduced outer diameter surface 321. Internal member 311 may be axially slid toward small forward aperture 305 to provide maximal extension of the elongate tapering snout 313 outside of the cylindrical housing 303. The firmness of the connection between the internal member 311 and the tube 33 will be sufficiently strong as to permit the internal member 311 to be axially manipulated by axially manipulating the tube 33.

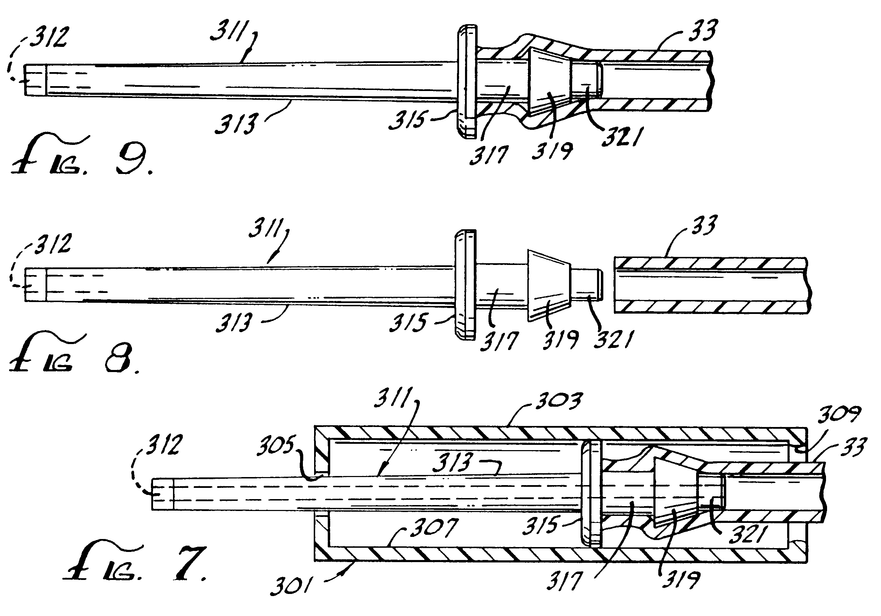

Connector 301 may be assembled at its point of use, as is illustrated in Figure 8. The internal member 311, available separately from the cylindrical housing 303 is first fitted with the tube 33 over its circular fitting 317, frusto-conical surface 319, and reduced outer diameter surface 321. By making the internal member 311 available separately, it is somewhat easier to fit the tube 33 onto the internal member 311. The end of tube 33 is pushed fully toward the external land 315 which assists in sealing the end of tube 33. Figure 9 illustrates the tube 33 in fully fitted position and against the external land 315.

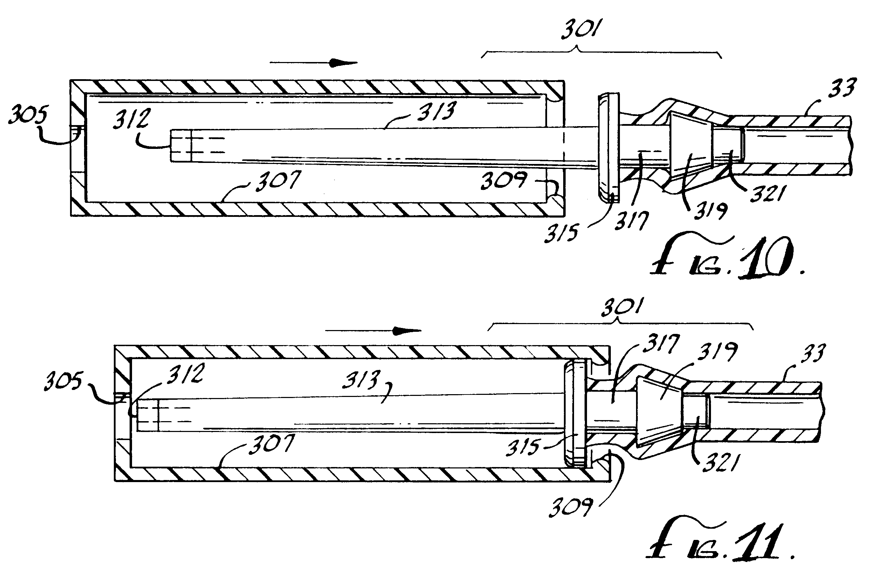

Referring to Figure 10, the internal member 311 is in position for insertion into the cylindrical housing 303. The edge of external land 315 of internal member 311 facing the circular inwardly disposed land 309 is rounded off, to facilitate the snap assembly of the internal member 311 past inwardly disposed land 309. The opposite edge of external land 315 is sharply terminated to prevent its movement past circular inwardly disposed land 309 and out of cylindrical housing 303.

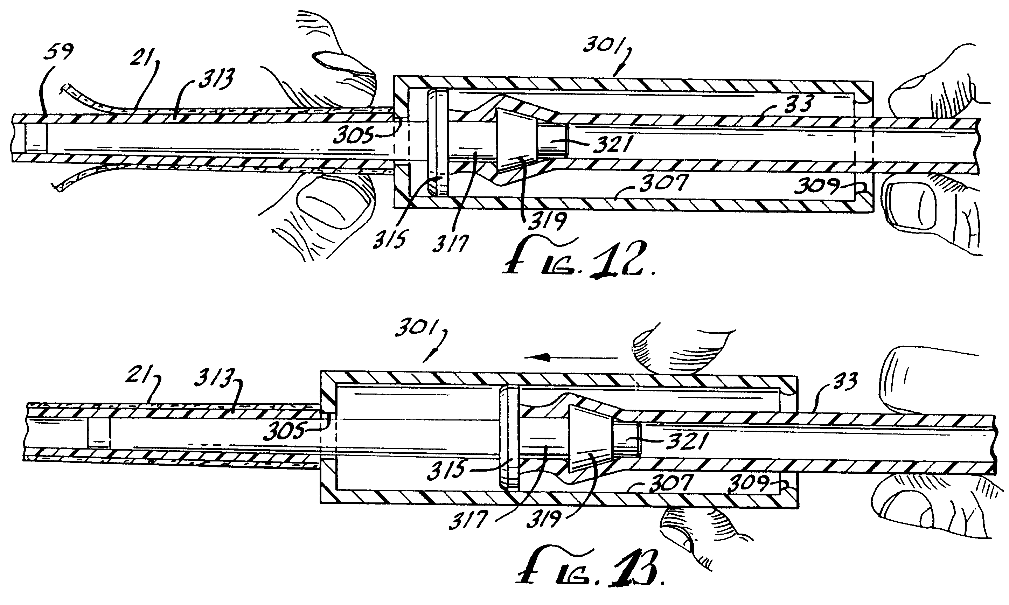

Depending upon the length of elongate tapering snout 313, or conversely, cylindrical housing 303, the end of elongate tapering snout 313 may have to be aligned with small forward aperture 305 before or after the external land 315 clears the circular inwardly disposed land 309. In any event, the elongate tapering snout 313 must extend far enough outside the boundary of cylindrical housing 303 to be effective. Figure 11 illustrates the connector 301 just after the snap fit insertion of the external land 315 past circular inwardly disposed land 309.

Referring to Figure 12, the connector 301 is illustrated in a position to connect with the perforated tube 59 of wick tube assembly 53. The generally upper aperture 61 and generally lower aperture 63 of perforated tube 59 are sealably engaged by a portion of the elongate tapering snout 313. The taper of elongate tapering snout 313 is, such as to allow easy insertion of the end of the elongate tapering snout 313 into the end of the perforated tube 59, and yet begin forming a seal with the internal surface of the perforated tube 59 once the end of the elongate tapering snout 313 is inserted about one-third of the way into the end of the perforated tube 59. The angle of taper and length of elongate tapering snout 313 may be adjusted depending upon the spacing of the generally upper aperture 61 and generally lower aperture 63 of perforated tube 59, and the distance from the open end of perforated tube 59 at which the wick tube assembly 53 begins. The other structures of pad 21 are numbered as per their appearance in the earlier figures.

To attach a pad 21, as is shown in Figure 12, the tube 33 is grasped in conjunction with the housing 303 to ensure that axial pressure on the elongate tapering snout 313 will not cause it to recede back into the housing 303. The dry pad is grasped with the other hand and pushed toward the elongate tapering snout 313 while the end of perforated tube 59 is axially slid over the elongate tapering snout 313, and until the end of perforated tube 59 abuts the exterior of the housing 303. At this point the pad 21 is fully connected to connector 301 and tube 33.

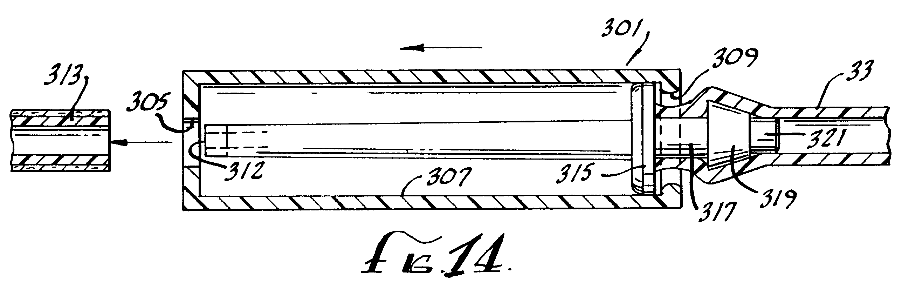

Figures 13 and 14 illustrate in sequence the ejection of a spent pad 21. Using the tube 33 and connector 301, the urine soaked pad 21 is lifted away from contact with the patient. The connector 301 with tube 33 and urine soaked pad 21 is positioned over a waste container. The connector 301 is grasped with one hand, while a section of the tube 33 adjacent the connector 301 is grasped with the other hand. The tube 33 is pulled axially away from the connector 301 causing the elongate tapering snout 313 to be withdrawn inside the housing 303. The end of the perforated tube 33 cannot be drawn inside the housing 303 since the diameter of the small forward aperture 305 is significantly smaller than the external diameter of the perforated tube 59. The elongate tapering snout 313 is then withdrawn from the end of the perforated tube 59 and into housing 303, as is shown in Figures 13 and 14, causing the urine soaked pad 21 to fall away into the waste container. In this manner, the attendant need not touch the urine soaked pad 21, and may dispose of same without exposure to an unsanitary object.

The operation of the improved pad and tube of the present invention has been described with respect to connector 301. The absorbent pad 21 of Figure 1 is placed near the urethral opening of the patient, especially a female patient whose urinary incontinence is to be managed. The non-sticking tear-away strip 45 may be removed from the adhesive layer 73, if the absorbent pad 21 is to be positioned against the patient and further secured with clothing or any other device having an appropriate inwardly disposed surface.

Vacuum pump 37 is started and the pressure in both the urine collection reservoir 35 and the tube 33 is lowered. Air within the absorbent pad 21 is drawn into apertures 61 and 63. Urine surrounding perforated tube 59 is thereby drawn into perforated tube 59.

A wicking path is formed for liquid urine to enter the first and second wicking materials 55 and 57 from any point along their surface areas and to be wicked within the planar portions of the wicking materials 55 and 57 in a direction toward perforated tube 59. Once the liquid urine is wicked into the portions of wicking materials 55 and 57 surrounding the perforated tube 59, they are available for entry into perforated tube 59 through the upper and lower apertures 61 and 63.

Liquid urine enters the apertures 61 and 63 due to the reduced pressure within perforated tube 59, reduced with respect to the ambient pressure of the surroundings and the ambient pressure of the urine in the wicking materials 55 and 57 at the opening of the upper and lower apertures 61 and 63. This pressure differential causes the aspiration action which causes the liquid urine to be aspirated into the perforated tube 59.

Once liquid urine enters perforated tube 59, it flows toward the tube-end 29 of the absorbent pad 21. The fluted void 67A assists in the transfer of the two-phase liquid urine-air flow as it moves through the tube 33. A tube 33 with a smooth profile, may, depending on the orientation of tube 33 with respect to gravity, create aspiration noise, such as gurgling, as the aspirating air becomes intermittently trapped behind blocking masses of liquid. The fluted void helps to provide a partially segregated path for the two-phase liquid urine-air flow, to avoid this problems and enhance flow efficiency.

The improved pad of the present invention, utilizable with a urine aspiration system having a vacuum pump and collection reservoir, utilizes a perforated tube within the volume of an absorbent pad which is surrounded by a wicking structure which wicks liquid away from the edges of the pad and towards the central perforated tube. The wicking structure frictionally engages the absorbent material forming the predominate volume of the pad to provide greater pad stability.

The connector engages the centrally located aspiration tube within the pad at a point near the end of the pad. The liquid sealing is performed about a face which has a plane generally perpendicular to the direction of flow.

Additionally, the connector snout is longer than the distance between the open end 29 and the point at which wick tube assembly 53 is encountered.

## Claims

1. An absorptive aspiratable pad (21) comprising an elongate fluid impermeable lower liner (71), defining a planar surface forming the bottom and at least a portion of the sides of the pad (21), bulk absorbent material (69) supported by and co-extensive with the impermeable lower liner (71) and covered by a layer of non-wetting permeable material (25), which substantially envelopes the lower liner (71), the bulk absorbent material (69) and an elongate tube (59) having a multiplicity of perforations situated within the permeable material (25) and in fluid contact with the bulk absorbent material (69), characterised in that a first layer of wicking material (55) is attached to the elongate tube (59) and extends substantially the length of and in contact with the bulk absorbent material (69) and that a second layer of wicking material (57) is attached to the elongate tube (59) and to the first layer of elongate wicking material (55), whereby the first and second layers (55, 57) of wicking material and the elongate tube (59) form a wicked tube assembly (53).

2. A pad as claimed in Claim 1 wherein the bulk absorbent material (69) substantially covers the length of the wicked tube assembly (53).

3. A pad as claimed in Claim 1 or 2 wherein the elongate tube (59) and the first and second layers (55, 57) of wicking material lie outside of and adjacent the bulk absorbent material (69) opposite the elongate fluid impermeable lower liner (71).

4. A pad as claimed in Claims 1 or 2 wherein the elongate tube (59) and the first and second layers (55, 57) of wicking material lie outside of and adjacent the bulk absorbent material (69) and adjacent the elongate fluid impermeable lower liner (71).

5. A pad as claimed in Claim 1 wherein the elongate tube (59) and the first and second layers (55, 57) of wicking material lie substantially within the bulk absorbent material (69).

## Patentansprüche

1. Aufnahmefähige, saugfähige Vorlage, umfassend eine längliche, für Flüssigkeit undurchlässige, untere Lage, welche eine ebene Fläche umreißt, die die Unterseite und mindestens einen Abschnitt der Seitenteile der Vorlage (21) bildet, loses saugfähiges Material (69), welches von der undurchlässigen unteren Lage (71) abgestützt wird und sich innerhalb dieser befindet, und durch eine Schicht von nichtbenetzungsfähigem, wasserdurchlässigem Material (25) abgedeckt wird, welches die untere Lage (71) umhüllt, wobei das lose saugfähige Material und ein längliches Rohr (59) eine Vielzahl von Lochungen aufweisen, und innerhalb des wasserdurchlässigen Materials (25) gelegen sind, und in Fluidverbindung zu dem losen saugfähigen Material (69) stehen, **dadurch gekennzeichnet**, daß eine erste Schicht aus kapillarem Material (55) an dem länglichen Rohr (59) befestigt ist und sich im wesentlichen entlang von dessen gesamter Länge erstreckt und Kontakt zu dem losen saugfähigen Material (69) hat, und daß eine zweite Schicht von kapillarem Material (57) an dem länglichen Rohr (59) und an der ersten Schicht des sich längs erstreckendem kapillaren Materials (55) befestigt ist, wodurch die erste und die zweite Schicht (55, 57) von kapillarem Material sowie das längliche Rohr (59) eine Kapillar-Rohreinheit (53) bilden.

2. Vorlage nach Anspruch 1, **dadurch gekennzeichnet**, daß das lose saugfähige Material (69) im wesentlichen die Länge der Kapillar-Rohreinheit (53) abdeckt.

3. Vorlage nach Anspruch 1 oder 2, **dadurch gekennzeichnet**, daß das längliche Rohr (59) und die erste und zweite Schicht (55, 57) des kapillaren Materials außerhalb des losen saugfähigen Materials (69) und an dieses angrenzend, gegenüber der für Flüssigkeit undurchlässigen unteren Lage (71), angeordnet sind.

4. Vorlage nach Anspruch 1 oder 2, **dadurch gekennzeichnet**, daß das längliche Rohr (59) und die erste und die zweite Schicht (55, 57) des kapillaren Materials außerhalb von und benachbart von dem losen saugfähigen Material (69) und benachbart zu der länglichen, für Flüssigkeit undurchlässigen unteren Lage (71) angeordnet sind.

5. Vorlage nach Anspruch 1, **dadurch gekennzeichnet**, daß das längliche Rohr (59) und die erste und zweite Schicht (55, 57) aus kapillarem Material im wesentlichen innerhalb des losen saugfähigen Materials (69) angeordnet sind.

## Revendications

1. Couche absorbante à aspiration (21) comprenant une doublure inférieure allongée imperméable aux fluides (71), définissant une surface plane formant le fond et au moins une partie des côtés de la couche (21), une matière absorbante de remplissage (69) supportée de manière coextensive par la doublure inférieure imperméable (71), et recouverte d'une feuille de matière perméable non mouillante (25) qui enveloppe essentiellement la doublure inférieure (71), la matière absorbante de remplissage (69) et un long tube (59) pourvu d'une pluralité de perforations situées à l'intérieur de la matière perméable (25) et en contact d'écoulement de fluide avec la matière absorbante de remplissage (69), caractérisée en ce qu'une première épaisseur de matière de méchage (55) est fixée au long tube (59) et s'étend essentiellement le long de la matière absorbante de remplissage (69), au contact de celle-ci, et en ce qu'une seconde épaisseur de matière de méchage (57) est fixée au long tube (59) et à la première épaisseur de matière de méchage oblongue (55), les première et seconde épaisseurs (55, 57) de matière de méchage et le long tube (59) constituant un ensemble de tube méché (53).

2. Couche suivant la revendication 1, dans laquelle la matière absorbante de remplissage (69) recouvre sensiblement l'ensemble de tube méché (53).

3. Couche suivant la revendication 1 ou 2, dans laquelle le long tube (59) et les première et seconde épaisseurs (55, 57) de matière de méchage sont disposés en dehors et à proximité de la matière absorbante de remplissage (69), à l'opposé de la doublure inférieure allongée imperméable aux fluides (71).

4. Couche suivant la revendication 1 ou 2, dans laquelle le long tube (59) et les première et seconde épaisseurs (55, 57) de matière de méchage sont disposés en dehors et à proximité de la matière absorbante de remplissage (69) ainsi qu'à proximité de la doublure inférieure allongée imperméable aux fluides (71).

5. Couche suivant la revendication 1, dans laquelle le long tube (59) et les première et seconde épaisseurs (55, 57) de matière de méchage sont disposés essentiellement à l'intérieur de la matière absorbante de remplissage (69).
